# EUROPÄISCHE PATENTSCHRIFT

(11) **EP 2 308 823 B1**
(45) Veröffentlichungstag und Bekanntmachung des Hinweises auf die Patenterteilung: **03.06.2015**
(21) Anmeldenummer: 10009853.2
(22) Anmeldetag: 17.09.2010
(51) Int. Cl.: C07C 67/08, C07C 67/56, C07C 69/28

(54) **Verfahren zur Herstellung von Polyolestern**
Method for manufacturing polyol esters
Procédé de fabrication d'esters de polyols

(30) Priorität: 08.10.2009 DE 102009048771
(43) Veröffentlichungstag der Anmeldung: 13.04.2011
(73) Patentinhaber: OXEA GmbH, 46147 Oberhausen (DE)
(72) Erfinder: Weber, Tonia, Dr., 64293 Darmstadt (DE); Borgmeier, Oliver, Dr., 41470 Neuss (DE); Frey, Guido, D., Dr., 64560 Riedstadt (DE); Strutz, Heinz, Dr., 47445 Moers (DE)

(56) Entgegenhaltungen:
- WO-A1-2011/042116
- US-A- 2 469 446
- US-A- 2 628 249

## Beschreibung

Die Erfindung betrifft ein Verfahren zur Herstellung von Polyolestern aus linearen oder verzweigten aliphatischen Monocarbonsäuren mit 3 bis 20 Kohlenstoffatomen und Polyolen durch Umsetzung der Ausgangsverbindungen in Gegenwart eines Adsorbens.

Ester mehrwertiger Alkohole, auch Polyolester genannt, finden in großem Umfang und in vielfältiger Weise Anwendung in der Technik, wie zum Beispiel als Weichmacher oder Schmiermittel. Durch die Auswahl geeigneter Ausgangsprodukte lassen sich die physikalischen Stoffeigenschaften, wie zum Beispiel Siedepunkt oder Viskosität gezielt einstellen, und den chemischen Eigenschaften, wie der Hydrolyseresistenz oder der Stabilität gegenüber einem oxidativen Abbau Rechnung tragen. Auch auf die Lösung konkreter anwendungstechnischer Probleme können Polyolester gezielt zugeschnitten werden. Ausführliche Übersichten über den Einsatz von Polyolestern finden sich zum Beispiel in Ullmann's Encyclopedia of Industrial Chemistry, 5. Auflage, 1985, VCH Verlagsgesellschaft, Vol. A1, Seiten 305-319; 1990, Vol. A15, Seiten 438-440 oder in Kirk Othmer, Encyclopedia of Chemical Technology, 3. Auflage, John Wiley & Sons, 1978, Vol. 1, Seiten 778-787; 1981, Vol. 14, Seiten 496-498.

Die Verwendung von Polyolestern als Schmierstoffe besitzt eine große technische Bedeutung und sie werden besonders für solche Anwendungsgebiete eingesetzt, in denen Schmierstoffe auf Mineralölbasis die gesetzten Anforderungen nur unvollständig erfüllen. Polyolester werden insbesondere als Turbinenmotoren- und Instrumentenöle benutzt. Polyolester für Schmiermittelanwendungen basieren häufig auf 1,3-Propandiol, 1,3-Butandiol, 1,4-Butandiol, 1,2-Hexandiol, 1,6-Hexandiol, Neopentylglykol, Trimethylolpropan, Pentaerythrit, 2,2,4-Trimethylpentan-1,3-diol, Glycerin oder 3(4),8(9)-Dihydroxymethyltricyclo[5.2.1.0^{2.6}]decan, auch als TCD-Alkohol DM bezeichnet, als Alkoholkomponente.

Auch als Weichmacher werden Polyolester in erheblichem Umfang verwendet. Weichmacher finden in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u. a. mit dem Ergebnis, dass seine mechanischen Eigenschaften optimiert, z. B. Formveränderungsvermögen, Elastizität, Festigkeit erhöht werden und die Härte verringert wird.

Um Weichmachern möglichst weite Anwendungsgebiete zu eröffnen, müssen sie eine Reihe von Kriterien erfüllen. Im Idealfall sollten sie geruchlos, farblos, licht-, kälte- und wärmebeständig sein. Überdies erwartet man, dass sie unempfindlich gegenüber Wasser, schwer brennbar und wenig flüchtig sind und die Gesundheit nicht schädigen. Weiterhin soll die Herstellung der Weichmacher einfach sein und, um ökologischen Anforderungen zu genügen, unter Vermeidung von Abfallstoffen, wie nicht weiterverwertbaren Nebenprodukten und Schadstoffe enthaltenden Abwässern, erfolgen. Eine spezielle Klasse von Polyolestern (sie werden kurz als G-Ester bezeichnet) enthält als Alkoholkomponente Diole bzw. Etherdiole, beispielsweise Ethylenglykol, Diethylenglykol, Triethylenglykol, Tetraethylenglykol, 1,2-Propylenglykol oder höhere Propylenglykole. Ihre Herstellung kann auf unterschiedlichen Wegen erfolgen. Neben der Umsetzung von Alkohol und Säure gegebenenfalls in Gegenwart saurer Katalysatoren werden in der Praxis weitere Prozesse zur Gewinnung von G-Estern angewandt, u. a. die Reaktion von Diol mit Säurehalogenid, die Umesterung eines Carbonsäureesters mit einem Diol und die Addition von Ethylenoxid an Carbonsäuren (Ethoxylierung). In der industriellen Fertigung haben sich lediglich die unmittelbare Umsetzung von Diol und Carbonsäure und die Ethoxylierung von Carbonsäuren als Produktionsverfahren durchgesetzt, wobei der Veresterung von Diol und Säure zumeist der Vorzug gegeben wird. Denn dieses Verfahren kann ohne besonderen Aufwand in konventionellen chemischen Apparaten durchgeführt werden und es liefert chemisch einheitliche Produkte. Demgegenüber erfordert die Ethoxylierung umfangreiche und kostenintensive technische Mittel. Ethylenoxid ist eine sehr aggressive chemische Substanz. Es kann explosionsartig polymerisieren und bildet mit Luft in sehr weiten Mischungsbereichen explosive Gemische. Ethylenoxid reizt die Augen und Atemwege, führt zu Verätzungen, zu Leber- und Nierenschäden und ist karzinogen. Seine Handhabung erfordert daher umfangreiche Sicherheitsmaßnahmen. Überdies ist auf peinliche Sauberkeit von Lagervorrichtungen und Reaktionsapparaten zu achten, um die Bildung unerwünschter Verunreinigungen durch Nebenreaktionen des Ethylenoxids mit Fremdstoffen auszuschließen. Schließlich ist die Reaktion mit Ethylenoxid nicht sehr selektiv, denn sie führt zu Gemischen von Verbindungen unterschiedlicher Kettenlänge.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuss und/oder die Abtrennung des im Verlauf der Reaktion gebildeten Wassers stellt sicher, dass das Gleichgewicht entsprechend dem Massenwirkungsgesetz zur Seite des Reaktionsproduktes, also des Esters, verschoben wird, d. h. hohe Ausbeuten erzielt werden.

Umfassende Angaben zur Herstellung von Estern mehrwertiger Alkohole, darunter auch Ester aus Ethylenglykolen und Fettsäuren und zu den Eigenschaften ausgewählter Vertreter dieser Verbindungsklassen finden sich in Goldsmith, Polyhydric Alcohol Esters of Fatty Acids, Chem. Rev. 33, 257 ff. (1943). Beispielsweise erfolgt die Herstellung von Estern des Diethylenglykols, des Triethylenglykols und von Polyethylenglykolen über Reaktionszeiten von 2,5 bis 8 Stunden bei Temperaturen von 130 bis 230°C. Zur Entfernung des Reaktionswassers verwendet man Kohlendioxid. Als geeignete Katalysatoren für die Veresterung mehrwertiger Alkohole werden anorganische Säuren, saure Salze, organische Sulfonsäuren, Acetylchlorid, Metalle oder amphotere Metalloxide genannt. Die Entfernung des Reaktionswassers erfolgt mit Hilfe eines Schleppmittels, beispielsweise Toluol oder Xylol oder durch Einleiten inerter Gase wie Kohlendioxid oder Stickstoff.

Auf die Gewinnung und die Eigenschaften von Fettsäureestern der Polyethylenglykole geht Johnson (Edit.), Fatty Acids in Industry (1989) Kap. 9, Polyoxyethylene Esters of Fatty Acid ein und gibt eine Reihe präparativer Hinweise. Höhere Diesterkonzentrationen erzielt man durch die Erhöhung des molaren Verhältnisses von Carbonsäure zu Glykol. Geeignete Maßnahmen zur Entfernung des Reaktionswassers sind die Azeotropdestillation in Gegenwart eines mit Wasser nicht mischbaren Lösungsmittels, das Erhitzen unter Durchleiten eines Inertgases oder die Durchführung der Reaktion unter Vakuum in Gegenwart eines Trockenmittels. Verzichtet man auf den Zusatz von Katalysatoren, so sind längere Reaktionszeiten und höhere Reaktionstemperaturen erforderlich. Beide Reaktionsbedingungen können durch den Einsatz von Katalysatoren gemildert werden. Neben Schwefelsäure sind organische Säuren wie p-Toluolsulfonsäure sowie Kationenaustauscher vom Polystyroltyp die bevorzugten Katalysatoren. Auch die Verwendung von Metallpulvern, wie Zinn oder Eisen, wird beschrieben. Nach der Lehre aus US 2,628,249 lassen sich Farbprobleme bei der Katalyse mit Schwefelsäure oder Sulfonsäuren mildern, wenn in Gegenwart von Aktivkohle gearbeitet wird.

WO 2011/042116 A1 betrifft die Herstellung von Polyolestern durch Umsetzung von Polyolen mit linearen oder verzweigten aliphatischen Monocarbonsäuren mit 3 bis 20 Kohlenstoffatomen in Gegenwart eines Lewissäurekatalysators mit nachfolgender Wasserdampfbehandlung. Die Umsetzung wird gegebenenfalls in Gegenwart eines Adsorbens durchgeführt.

Eine Arbeitsweise, bei der Ester des Diethylen- und Triethylenglykols und der Caprylsäure ohne Katalysatorzusatz hergestellt werden, ist aus US 2,469,446 bekannt. Die Veresterungstemperatur liegt im Bereich von 270 bis 275°C und das Reaktionswasser wird mittels eines Kohlendioxidstromes ausgetrieben.

Bei der Reaktionsführung, bei der man auf die Zugabe eines Katalysators verzichtet, arbeitet man im Allgemeinen mit einem molaren Überschuss an der jeweiligen Carbonsäure, die aufgrund ihrer Acidität auch als Katalysator wirkt.

Für die Abtrennung des bei der Esterbildung aus dem Polyol und der Carbonsäuren gebildeten Reaktionswassers sind verschiedene Verfahren bekannt. Beispielsweise wird das gebildete Reaktionswasser zusammen mit der überschüssigen Carbonsäure aus dem Reaktionsgefäß abdestilliert und in einen nachgeschalteten Phasentrenner geleitet, in dem sich Carbonsäure und Wasser je nach ihren Löslichkeitseigenschaften auftrennen. Gegebenenfalls bildet die eingesetzte Carbonsäure mit Wasser unter den Reaktionsbedingungen auch ein Azeotrop und vermag als Schleppmittel das Reaktionswasser zu entfernen. Anwendung findet auch die Azeotropdestillation in Gegenwart eines zugesetzten mit Wasser nicht mischbaren Lösemittels, das Erhitzen des Reaktionsgemisches unter Durchleiten eines inerten Gases, die Umsetzung der Ausgangsstoffe Polyol und Carbonsäure unter Vakuum oder in Gegenwart eines Trocknungsmittels. Insbesondere die Wasserentfernung durch azeotrope Destillation hat sich für die Einstellung des Gleichgewichts bei der Herstellung von Polyolestern bewährt. Nach der aus DE 199 40 991 A1 bekannten Arbeitsweise wird das mit Wasser nicht mischbare Lösemittel, das als Schleppmittel wirkt und das einen Siedepunkt von weniger als 112°C aufzuweisen hat, dem Reaktionsansatz erst bei Erreichen einer Temperatur von mindestens 140°C zugesetzt.

Der nach Abtrennung des Reaktionswassers und überschüssiger, nicht umgesetzter Ausgangsstoffe, zweckmäßigerweise die im Überschuss zugesetzte Carbonsäure, angefallene Rohester, kann zunächst mit einem alkalischen Reagenz, zum Beispiel mit einer wässrigen Soda- oder Natriumhydroxidlösung behandelt werden, um letzte Reste acider Bestandteile zu entfernen. Nach Wasserwäsche, Behandlung mit Bleicherde und Aktivkohle kann zur Entfernung letzter Spuren von farb- und geruchsgebenden Stoffen Vakuum bei erhöhter Temperatur angelegt werden. Aufarbeitungsverfahren von rohen Polyolestern sind beispielsweise aus US 2,469,446 A1 bekannt. Gegebenenfalls ist die Behandlung mit Bleichmitteln und Aktivkohle mehrmals zu wiederholen, um Endprodukte mit zufriedenstellenden Farbeigenschaften zu gewinnen. Gemäß der aus DE 199 40 991 A1 bekannten Arbeitsweise wird der Rohester nach Alkalibehandlung getrocknet, beispielsweise indem man ein inertes Gas durch das Produkt leitet oder Vakuum anlegt und gegebenenfalls noch zusätzlich im Vakuum destilliert. Zur Farbverbesserung von Polyolestern schlägt WO 94/18153 A1 eine nachträgliche Behandlung mit einer wässrigen Wasserstoffperoxidlösung vor.

Wegen der eingangs beschriebenen Qualitätskriterien für Polyolester sind die Verfahrensschritte bei der Veresterungsstufe unter Entfernung des Reaktionswassers und bei der Aufarbeitung des Rohesters sehr wesentliche Prozessmerkmale, denn die Abstimmung dieser Verfahrensschritte beeinflusst in wesentlichem Maße die sensorischen und optischen Eigenschaften der Endprodukte. Insbesondere werden hohe Anforderungen an die Farbeigenschaften, wie geringe Farbzahl und hohe Farbstabilität, der Polyolester gestellt. Die Struktur der Ausgangsstoffe, der mehrwertigen Alkohole und der Säuren, ist dagegen für die mechanischen und thermischen Eigenschaften der mit den Polyolestern weichgestellten Kunststoffmassen maßgebend und beeinflusst die Hydrolyse- und Oxidationsstabilität von Schmiermitteln.

Es wurde nun überraschenderweise gefunden, dass sich Polyolester aus Polyolen und linearen oder verzweigten aliphatischen Monocarbonsäuren mit einer ausgezeichneten Farbzahl und Farbstabilität herstellen lassen, wenn die Veresterungsreaktion in Gegenwart eines Adsorbens erfolgt und bei der anschließenden Aufarbeitung des Rohprodukts eine Wasserdampfbehandlung durchgeführt wird.

Die Erfindung besteht daher in einem Verfahren zur Herstellung von Polyolestern durch Umsetzung von Polyolen der allgemeinen Formel

H-(-O-[-CR¹R²-]ₘ-)ₒ-OH

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxylmethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, mit linearen oder verzweigten aliphatischen Monocarbonsäuren mit 3 bis 20 Kohlenstoffatomen im Überschuss und anschließende Aufarbeitung des Reaktionsgemisches. Es ist dadurch gekennzeichnet, dass man eine Mischung der Ausgangsverbindungen in Gegenwart eines Adsorbens reagieren lässt, die unumgesetzten Ausgangsverbindungen abtrennt, anschließend eine Wasserdampfbehandlung bei einer Temperatur von 170 bis 220°C und über einen Zeitraum von 0,5 bis 5 Stunden durchführt und den zurückgebliebenen Polyolester trocknet, wobei nach beendeter Umsetzung oder nach einer beliebigen anderen Aufarbeitungsmaßnahme der Polyolester filtriert wird, mit der Maßgabe, dass die aliphatische Monocarbonsäure einen niedrigeren Siedepunkt als das eingesetzte Polyol aufweist und die Umsetzung ohne Einsatz eines Katalysators autokatalytisch durchgeführt wird.

Die neue Arbeitsweise zeichnet sich durch große Zuverlässigkeit nicht nur im Labor- und Versuchsbetrieb sondern vor allem auch in technischen Anlagen aus. Sie ist, auch kontinuierlich, leicht durchzuführen und liefert Polyolester mit hoher Reinheit. Die Anwesenheit eines Adsorbens in der Veresterungsstufe, verbunden mit der Wasserdampfbehandlung in der Aufarbeitungsstufe, ist für das erfindungsgemäße Verfahren von entscheidender Bedeutung und führt zu ausgezeichneten Farbeigenschaften und bemerkenswerter Farbstabilität von Polyolestern.

Als Adsorbentien, die während der Veresterungsreaktion zugegen sind, verwendet man poröse, großflächige feste Materialien, die üblicherweise in der chemischen Praxis sowohl im Labor als auch in technischen Anlagen eingesetzt werden. Beispiele für solche Materialien sind oberflächenreiche Polykieselsäuren wie Silicagele (Kiesel-Xerogele), Kieselgel, Kieselguhr, oberflächenreiche Aluminiumoxide und Aluminiumoxidhydrate, mineralische Materialien, wie Tone, Carbonate oder Aktivkohle. Besonders bewährt hat sich Aktivkohle. Im Allgemeinen ist das Adsorbens feinteilig in der Reaktionslösung suspendiert, die durch intensives Rühren oder durch Einleiten eines Inertgases bewegt wird. Dadurch wird ein inniger Kontakt zwischen der flüssigen Phase und dem Adsorbens erreicht. Das Massenverhältnis von flüssiger Phase zu Adsorbens kann weitgehend frei und somit den individuellen Erfordernissen entsprechend eingestellt werden. Es hat sich bewährt, je 100 Gew.-Teile flüssiger Phase 0,05 bis 30, vorzugsweise 0,1 bis 5,0 und insbesondere 0,1 bis 1,0 Gew.-Teile Adsorbens einzusetzen. Nach beendeter Umsetzung kann das Adsorbens aus dem Prozess abgetrennt und in das Veresterungsgefäß zurückgeführt und wieder eingesetzt werden. Der Wiedereinsatz ist sooft möglich, bis die Entfärbekraft des Adsorbens erschöpft ist. Es ist aber auch möglich, das Adsorbens in dem Rohprodukt zu belassen und es während des Aufarbeitungsverfahrens an einer beliebigen aber zweckmäßigen Stufe abzutrennen.

Die Reaktion von Polyolen und aliphatischen Monocarbonsäuren wird ohne Einsatz eines Katalysators durchgeführt. Diese Variante der Umsetzung hat den Vorteil, dass man vermeidet, dem Reaktionsgemisch Fremdstoffe zuzuführen, die zu einer unerwünschten Verunreinigung des Polyolesters führen kann. Allerdings muss man dann im Allgemeinen höhere Reaktionstemperaturen einhalten, weil nur so gewährleistet ist, dass die Umsetzung mit ausreichender, d. h. wirtschaftlich vertretbarer Geschwindigkeit abläuft. Zu beachten ist in diesem Zusammenhang, dass die Steigerung der Temperatur zu einer thermischen Schädigung des Polyolesters führen kann. Der Überschuss der aliphatischen Monocarbonsäure, die gleichzeitig Reaktionskomponente des Polyols ist, wirkt als Katalysator, so dass die Reaktion autokatalytisch abläuft.

Man lässt das Polyol mit überschüssiger Monocarbonsäure reagieren und ohne Zugabe eines Katalysators, so dass die überschüssige Monocarbonsäure selbst als Katalysator wirkt. Auch lässt sich überschüssige Monocarbonsäure, die einen niedrigeren Siedepunkt als das eingesetzte Polyol aufweist, auf einfache Weise destillativ aus dem Rohester abtrennen. Die aliphatische Monocarbonsäure wird in einem 10 bis 50%-igen molaren, vorzugsweise in einem 20 bis 40%-igen molaren Überschuss je Mol zu veresternder Hydroxylgruppe des Polyols eingesetzt.

Das gebildete Reaktionswasser wird im Laufe der Reaktion zusammen mit der überschüssigen Monocarbonsäure aus dem Reaktionsgefäß abdestilliert und in einen nachgeschalteten Phasentrenner geleitet, in dem sich die Monocarbonsäure und Wasser je nach ihren Löslichkeitseigenschaften auftrennen. Gegebenenfalls bildet die eingesetzte Monocarbonsäure mit Wasser unter den Reaktionsbedingungen auch ein Azeotrop und vermag als Schleppmittel das Reaktionswasser zu entfernen. Aus dem Wasseranfall kann der Reaktionsverlauf verfolgt werden. Das abgeschiedene Wasser wird aus dem Prozess entfernt während die Monocarbonsäure aus dem Phasentrenner wieder in das Reaktionsgefäß zurückfließt. Die Zugabe eines weiteren organischen Lösungsmittels, wie Hexan, 1-Hexen, Cyclohexan, Toluol, Xylol oder Xylolisomerengemische, das die Aufgabe des Azeotropbildners übernimmt, ist nicht ausgeschlossen, jedoch auf wenige Ausnahmefälle beschränkt. Der Azeotropbildner kann bereits zu Beginn der Veresterungsreaktion oder nach Erreichen höherer Temperaturen zugesetzt werden. Wenn die theoretisch zu erwartende Wassermenge angefallen ist oder die Hydroxylzahl, beispielsweise bestimmt nach DIN 53240, unter einen festgelegten Wert gefallen ist, beendet man die Reaktion indem man den Reaktionsansatz abkühlen lässt.

Die Reaktion zwischen Polyol und aliphatischer Monocarbonsäure setzt in Abhängigkeit von den Einsatzmaterialien im Bereich von etwa 120 bis 180°C ein und kann auf unterschiedlich ausgestaltete Weise zu Ende geführt werden.

Nach einer Ausgestaltung des erfindungsgemäßen Verfahrens wird zunächst ausgehend von Raumtemperatur auf eine Temperatur bis auf maximal 280°C, vorzugsweise bis auf maximal 250°C erhitzt und bei konstant gehaltener Temperatur der Druck ausgehend von Normaldruck stufenweise erniedrigt, um die Entfernung des Reaktionswassers zu erleichtern. Die Wahl der Druckstufen, ob ein-, zwei- oder mehrstufig, sowie der auf der jeweiligen Stufe einzustellende Druck kann über einen weiten Bereich variiert und den jeweiligen Bedingungen angepasst werden. Beispielsweise kann in einer ersten Stufe der Druck ausgehend von Normaldruck zunächst bis auf 600 hPa erniedrigt werden und anschließend die Reaktion bei einem Druck von 300 hPa zu Ende geführt werden. Bei diesen Druckangaben handelt es sich um Richtwerte, die zweckmäßig eingehalten werden.

Neben der Variation des Drucks kann ebenfalls auch die Temperatur ausgehend von Raumtemperatur während der Veresterungsreaktion ein-, zwei- oder mehrstufig verändert werden, so dass bei konstant eingestelltem Druck die Temperatur von Stufe zu Stufe erhöht wird, üblicherweise bis auf eine maximale Temperatur von 280°C. Es hat sich aber als zweckmäßig erwiesen, bei von Stufe zu Stufe ansteigender Temperatur auf maximal 280°C zu erhitzen und auch den Druck von Stufe zu Stufe zu erniedrigen. Beispielsweise kann die Veresterungsreaktion ausgehend von Raumtemperatur in einer ersten Stufe bei einer Temperatur bis auf 190°C geführt werden. Ebenfalls wird ein verminderter Druck bis auf 600 hPa angelegt, um das Austreiben des Reaktionswassers zu beschleunigen. Nach Erreichen der Temperaturstufe von 190°C wird der Druck nochmals bis auf 300 hPa erniedrigt und die Veresterungsreaktion bei einer Temperatur bis auf 250°C zu Ende geführt. Bei diesen Temperatur- und Druckangaben handelt es sich um Richtwerte, die zweckmäßiger Weise eingehalten werden. Die einzustellenden Temperatur- und Druckbedingungen auf den jeweiligen Stufen, die Zahl der Stufen sowie die jeweilige Temperaturerhöhungs- oder Druckminderungsrate pro Zeiteinheit können über einen weiten Bereich variiert werden und entsprechend den physikalischen Eigenschaften der Ausgangsverbindungen und der Reaktionsprodukte angepasst werden, wobei die Temperatur- und Druckbedingungen der ersten Stufe ausgehend vom Normaldruck und Raumtemperatur eingestellt werden. Besonders zweckmäßig hat es sich erwiesen, die Temperatur in zwei Stufen zu erhöhen und den Druck in zwei Stufen zu erniedrigen.

Die untere Grenze des einzustellenden Drucks hängt von den physikalischen Eigenschaften, wie Siedepunkte und Dampfdrücke, der Ausgangsverbindungen sowie der gebildeten Reaktionsprodukte ab und wird auch durch die Anlagenausstattung festgelegt. Ausgehend vom Normaldruck kann innerhalb dieser Grenzwerte stufenweise mit von Stufe zu Stufe abnehmenden Drücken gearbeitet werden. Die obere Temperaturgrenze, üblicherweise 280°C, ist einzuhalten, um die Bildung von Zersetzungsprodukten, die u. a. farbschädigend wirken, zu vermeiden. Die untere Grenze der Temperaturstufen wird durch die Reaktionsgeschwindigkeit bestimmt, die noch ausreichend hoch sein muss, um die Veresterungsreaktion innerhalb einer vertretbaren Zeit abzuschließen. Innerhalb dieser Grenzwerte kann stufenweise mit von Stufe zu Stufe ansteigenden Temperaturen gearbeitet werden.

Das nach Beendigung der Umsetzung anfallende Reaktionsgemisch enthält neben dem Polyolester als erwünschtem Reaktionsprodukt nicht umgesetzte Ausgangsstoffe, insbesondere noch überschüssige aliphatische Monocarbonsäure, da nach dem erfindungsgemäßen Verfahren mit einem Monocarbonsäureüberschuss gearbeitet wird. Üblicherweise destilliert man zunächst unumgesetzte und im Überschuss vorliegende Ausgangsverbindungen ab, zweckmäßigerweise unter Anlegen eines verminderten Drucks.

Die Aufarbeitung des erhaltenen Rohesters kann in unterschiedlicher Weise ausgestaltet werden. So kann zunächst der Rohester vom anwesenden Adsorbens befreit werden. Man filtriert das Produkt in üblichen Filtrierapparaten bei normaler Temperatur oder bei Temperaturen bis 150°C. Die Filtration kann durch gängige Filterhilfsmittel, wie Zellulose, Kieselgel, Kieselgur, Holzmehl, unterstützt werden. Die Abtrennung des Adsorbens aus dem rohen Veresterungsprodukt ist jedoch nicht zwingend erforderlich und die weitere Aufarbeitung kann in Gegenwart oder in Abwesenheit des Adsorbens erfolgen.

Im Anschluss wird der, gegebenenfalls mit Alkali behandelte oder filtrierte, Rohester einer Behandlung mit Wasserdampf unterzogen, die beispielsweise in einfacher Form durch Einleiten von Wasserdampf in das Rohprodukt erfolgen kann. Auch trägt die Wasserdampfbehandlung zur Farbzahlverbesserung des Polyolesters bei. Ist noch das während der Veresterungsreaktion zugesetzte Adsorbens zugegen, wirkt sich die Anwesenheit eines Adsorbens während der Wasserdampfbehandlung ebenfalls vorteilhaft auf die Farbe und auf die Farbstabilität des Polyolesters aus und seine Gegenwart ist daher zu empfehlen. Es ist aber auch möglich, nach beendeter Veresterungsreaktion und Abtrennung überschüssiger Ausgangsverbindungen, also vor Durchführung der Wasserdampfdestillation, das Adsorbens abzufiltrieren.

Die Wasserdampfbehandlung wird im Allgemeinen bei Normaldruck durchgeführt, obwohl die Anwendung eines leichten Unterdrucks zweckmäßigerweise bis 400 hPa nicht ausgeschlossen ist. Die Wasserdampfbehandlung erfolgt bei Temperaturen von 170 bis 220°C, vorzugsweise von 170 bis 200°C und richtet sich auch nach den physikalischen Eigenschaften der jeweils herzustellenden Polyolester.

Bei dem Prozessschritt der Wasserdampfbehandlung erweist es sich als zweckmäßig, während der Aufheizperiode bis zum Erreichen der Arbeitstemperatur möglichst schonend vorzugehen, um den Rohester auf die erforderliche Temperatur für die Wasserdampfbehandlung zu erhitzen.

Die Dauer der Wasserdampfbehandlung lässt sich durch Routineversuche ermitteln und sie wird über einen Zeitraum von 0,5 bis 5 Stunden durchgeführt. Eine zu lange Wasserdampfbehandlung führt zu einer ungewünschten Erhöhung der Farbzahl des Polyolesters und ist daher zu vermeiden. Auch beobachtet man eine verstärkte Abbaureaktion des Polyolesters zu sauer reagierenden Verbindungen, deren Gehalt sich in einem Anstieg der Neutralisationszahl oder Säurezahl, beispielsweise bestimmt nach DIN EN ISO 3682/ASTM D 1613, zeigt. Bei einer zu kurzen Behandlungsdauer ist die Entfernung von Resten der Ausgangsverbindungen und des Wassers nicht vollständig und der gewünschte Polyolester weist noch eine zu hohe unerwünschte Säurezahl und einen zu hohen Wassergehalt auf. Auch wird bei zu kurzer Behandlungsdauer nur ein geringer vorteilhafter Effekt auf die Farbzahl des Polyolesters beobachtet.

Die Bedingungen der Wasserdampfbehandlung, wie Temperatur, Druck und Dauer sind gezielt auf den jeweiligen Polyolester einzustellen, um ein optimales Ergebnis in Bezug auf die Farbzahl des Polyolesters zu erzielen und um Restgehalte an Ausgangsverbindungen und Wasser möglichst zu minimieren und gleichzeitig Abbaureaktionen zu unterdrücken.

Insbesondere bei Polyolestern auf Basis von Etherdiolen, wie zum Beispiel Triethylenglykol oder Tetraethylenglykol, sind die Bedingungen bei der Wasserdampfbehandlung auf den jeweiligen Polyolester zuzuschneiden, um den unerwünschten Abbau der Etherkette zu unterbinden.

An die Wasserdampfbehandlung schließt sich, gegebenenfalls nach Filtration des Adsorbens, die Trocknung des Polyolesters an, beispielsweise in dem man ein inertes Gas durch das Produkt bei erhöhter Temperatur leitet. Es kann auch bei erhöhter Temperatur gleichzeitig ein Unterdruck angelegt werden und gegebenenfalls ein inertes Gas durch das Produkt geleitet werden. Auch ohne Einwirken eines inerten Gases kann nur bei erhöhter Temperatur oder nur bei geringerem Druck gearbeitet werden. Die jeweiligen und auf den jeweiligen Polyolester zugeschnittenen Trocknungsbedingungen, wie Temperatur, Druck und Dauer lassen sich durch einfache Vorversuche ermitteln. Im Allgemeinen arbeitet man bei Temperaturen im Bereich von 80 bis 250°C, vorzugsweise 100 bis 180°C und bei Drücken von 0,2 bis 500 hPa, vorzugsweise 1 bis 200 hPa und insbesondere 1 bis 20 hPa. Falls noch das in der Veresterungsstufe zugesetzte Adbsorbens zugegen ist, wird der Polyolester filtriert. Die Filtration erfolgt in konventionellen Filtrierapparaturen bei normaler Temperatur oder bei Temperaturen bis 120°C. Die Filtration kann durch gängige Filtrierhilfsmittel wie Cellulose, Kieselgel, Kieselgur, Holzmehl, unterstützt werden. Ihr Einsatz ist jedoch auf Ausnahmefälle beschränkt.

Unabhängig davon, ob das in der Veresterungsstufe eingesetzte Adsorbens bereits zu Beginn der Aufarbeitungsmaßnahme abfiltriert wurde oder nach der Trocknung, kann es sich als zweckmäßig erweisen, den Polyolester einer nochmaligen Nachbehandlung mit einem Adsorbens zu unterziehen. Für diese Nachbehandlung eignen sich ebenfalls solche Adsorbentien, die auch in der Veresterungsstufe eingesetzt werden, insbesondere Aktivkohle. Abschließend wird dann der Polyolester nochmals filtriert.

Durch die Maßnahme, die Veresterungsreaktion in Gegenwart eines Adsorbens durchzuführen, werden bereits hellfarbige Rohprodukte erhalten, deren Farbqualität durch die in dem Aufarbeitungsschritt durchgeführte Wasserdampfbehandlung nochmals verbessert werden kann. Eine zusätzliche Behandlung des Polyolesters mit einer wässrigen Wasserstoffperoxid-Lösung zur Farbverbesserung ist nicht ausgeschlossen, aber nur auf Sonderfälle beschränkt.

Es werden hellfarbige Polyolester erhalten, die auch den übrigen Spezifikationen, wie Wassergehalt, Restsäuregehalt, Restgehalt an Katalysatorbestandteilen und Restgehalt an Monoester genügen.

Die als Ausgangsstoffe für das erfindungsgemäße Verfahren eingesetzten mehrwertigen Alkohole oder Polyole genügen der allgemeinen Formel

H-(-O-[-CR¹R²-]ₘ-)ₒ-OH

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

Als Polyole, die nach dem erfindungsgemäßen Verfahren zu hellfarbigen Polyolestern umgesetzt werden können, eignen sich beispielsweise Di-Trimethylolpropan oder Di-Pentaerythrit.

Als weitere Polyole kommen die Oligomeren von Ethylenglykol und 1,2-Propylenglykol, insbesondere die Etherdiole Di-, Tri- und Tetraethylenglykol oder Dipropylenglykol, Tripropylenglykol oder Tetrapropylenglykol in Betracht. Ethylen- und Propylenglykole sind industriell produzierte Chemikalien. Basissubstanz zu ihrer Herstellung ist Ethylenoxid und Propylenoxid, aus dem man 1,2-Ethylenglykol und 1,2-Propylenglykol durch Erhitzen mit Wasser unter Druck gewinnt. Diethylenglykol erhält man durch Ethoxylierung aus Ethylenglykol. Triethylenglykol fällt, wie Tetraethylenglykol, als Nebenprodukt bei der Hydrolyse von Ethylenoxid zur Herstellung von Ethylenglykol an. Beide Verbindungen können auch durch Umsetzung von Ethylenglykol mit Ethylenoxid synthetisiert werden. Dipropylenglykol, Tripropylenglykol, Tetrapropylenglykol und höhere Propoxylierungsprodukte sind aus der mehrfachen Addition von Propylenoxid an 1,2-Propylenglykol zugänglich.

Zur Gewinnung von hellfarbigen Polyolestern nach dem erfindungsgemäßen Prozess setzt man lineare oder verzweigte, aliphatische Monocarbonsäuren mit 3 bis 20 Kohlenstoffatomen im Molekül ein. Obgleich man gesättigten Säuren in vielen Fällen den Vorzug gibt, können, in Abhängigkeit vom jeweiligen Anwendungsgebiet der Weichmacher oder Schmiermittel, auch ungesättigte Carbonsäuren als Reaktionskomponente zur Estersynthese verwendet werden. Beispiele für Monocarbonsäuren als Bausteine von Polyolestern sind Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, Cyclohexancarbonsäure, 2-Ethylhexansäure, n-Nonansäure, 2-Methyloctansäure, Isononansäure, 3,5,5-Trimethylhexansäure, 2-Propylheptansäure, 2-Methylundecansäure, Isoundecancarbonsäure, Tricyclodecancarbonsäure und Isotridecan-carbonsäure. Besonders bewährt hat sich das neue Verfahren für die Herstellung von Polyolestern der oligomeren Ethylenglykole sowie der oligomeren Propylenglykole mit C₄- bis C₁₃- bzw. C₅- bis C₁₀-Monocarbonsäuren sowie zur Herstellung von Polyolestern auf Basis von Di-Trimethylolpropan.

Die Polyolester der Oligomeren des Ethylenglykols eignen sich hervorragend als Weichmacher für alle gängigen hochpolymeren thermoplastischen Stoffe. Besonders bewährt haben sie sich als Zusatz zu Polyvinylbutyral, das mit Glykolestern versetzt als Zwischenschicht zur Herstellung von Mehrschichten- oder Verbundgläsern verwendet wird. Sie können ebenfalls als Koaleszenzmittel oder Filmbildehilfsmittel in wässrigen Dispersionen von Kunststoffen, die als Beschichtungsmittel vielfältige Anwendung finden, eingesetzt werden. Nach dem erfindungsgemäßen Herstellungsverfahren lassen sich auf einfache Weise Polyolester mit ausgezeichneten Farbeigenschaften herstellen, die auch weiteren Qualitätsansprüchen, wie geringem Geruch oder einer geringen Säurezahl genügen. Besonders eignet sich das erfindungsgemäße Verfahren zur Herstellung von Triethylenglykol-di-2-ethyl-hexanoat (3G8 Ester), Tetraethylenglykol-di-n-heptanoat (4G7 Ester), Triethylenglykol-di-2-ethylbutyrat (3G6 Ester), Triethylenglykol-di-n-heptanoat (3G7 Ester) oder Tetraethylenglykol-di-2-ethylhexanoat (4G8 Ester).

Das erfindungsgemäße Verfahren kann kontinuierlich oder absatzweise in den für die chemische Technik typischen Reaktionsapparaten durchgeführt werden. Bewährt haben sich Rührkessel oder Reaktionsrohre, wobei die absatzweise Reaktionsführung die bevorzugte ist.

In den folgenden Beispielen wird das erfindungsgemäße Verfahren näher erläutert, es ist jedoch nicht auf die beschriebene Ausführungsform beschränkt.

### Ausführungsbeispiele:

### Beispiel 1:

### Herstellung von Triethylenglykol-di-2-ethylhexanoat (3G8 Ester); Veresterung in Gegenwart von Aktivkohle

Die Veresterung von Triethylenglykol mit 2-Ethylhexansäure wurde in einem beheizbaren 1 l - Vierhalskolben durchgeführt, der mit Rührer, Innenthermometer und einem Wasserabscheider ausgestattet wurde.

In dem Kolben wurden 250 Gramm (1,66 mol) Triethylenglykol und 680 Gramm (4,72 mol) 2-Ethylhexansäure sowie 0,4 Gew.-% Aktivkohle, bezogen auf den gesamten Reaktionsansatz, vorgelegt. Unter Rühren und unter Anlegen eines leichten Unterdrucks von 900 hPa wurde der Ansatz auf 225°C erhitzt. Bei Erreichen dieser Temperatur wurde der Druck schrittweise auf 400 hPa reduziert und gebildetes Reaktionswasser an dem Wasserabscheider abgenommen. Der Reaktionsverlauf wurde durch kontinuierliche Auswaage der über den Wasserabscheider ausgeschleusten Wassermenge sowie durch den Verlauf der Hydroxylzahl verfolgt. Nach insgesamt 14,5 Stunden Reaktionszeit wurde bei einer Resthydroxylzahl von 4,2 mg KOH/g (nach DIN 53240) die Reaktion beendet.

Anschließend wurde über einen Zeitraum von 3,75 Stunden bei einer Temperatur von 200°C und bei einem Druck von 20 hPa die überschüssige 2-Ethylhexansäure abdestilliert. Es folgte eine Wasserdampfdestillation über einen Zeitraum von 2,5 Stunden bei 200°C und bei Normaldruck. Nach abschließender Filtration von der Aktivkohle erhielt man hellfarbiges Triethylenglykol-di-2-ethylhexanoat mit den in der Tabelle 1 angegebenen Kennzahlen.

### Beispiel 2 (Vergleichsbeispiel):

### Herstellung von Triethylenglykol-di-2-ethylhexanoat (3G8 Ester); Veresterung ohne Zusatz von Aktivkohle

Es wurde gemäß dem Ausführungsbeispiel 1 gearbeitet, jedoch wurde die Veresterungsreaktion ohne Zusatz von Aktivkohle durchgeführt. Da keine Feststoffe zugegen waren, wurde der Ansatz nach Veresterung und Aufarbeitung nicht filtriert. Die gefundenen Kennzahlen sind ebenfalls in der nachfolgenden Tabelle aufgeführt.

**Tabelle 1: Kennzahlen von Triethylenglykol-di-2-ethylhexanoat, hergestellt nach den Beispielen 1 und 2**

| Gaschromatographische Analyse (Gew.-%): | | |
|---|---|---|
| | Beispiel 1 % | Beispiel 2 % |
| Triethylenglykol-di-2-ethylhexanoat | 97,5 | 97,7 |
| Triethylenglykol-mono-2-ethylhexanoat | 1,3 | 1,3 |
| Diethylenglykol-di-2-ethylhexanoat | 0,2 | 0,1 |
| Rest | 1,0 | 0,9 |

### Kennzahlen:

| | Beispiel 1 | Beispiel 2 |
|---|---|---|
| Farbzahl nach Hazen (DIN ISO 6271) | 24 | 173 |
| Neutralisationszahl (mg KOH/g, DIN EN ISO 3682/ ASTM D 1613) | 0,05 | 0,04 |
| Wassergehalt (Gew.-%, DIN 51777 Teil 1) | 0,02 | 0,01 |
| Hydroxylzahl (mg KOH/g; DIN 53240) | 2,9 | 2,2 |

Wie die Versuchsdaten der Tabelle 1 zeigen, ist der Zusatz von Aktivkohle in der Veresterungsstufe erforderlich, um hellfarbige Polyolester zu erhalten. Sowohl der Aktivkohlezusatz in der Veresterungsstufe wie auch die Wasserdampfdestillation während der Aufarbeitung sind notwendig, um hellfarbige Polyolester zu gewinnen. Eine alleinige Wasserdampfdestillation reicht für ein spezifikationsgerechtes Produkt nicht aus.

### Beispiel 3 (Vergleichsbeispiel):

### Herstellung von Neopentylglykol-di-2-ethylhexanoat, Veresterung in Gegenwart von Aktivkohle

Die Veresterung von Neopentylglykol mit 2-Ethylhexansäure wurde in einem beheizbaren 1 l- Vierhalskolben durchgeführt, der mit Rührer, Innenthermometer und einem Wasserabscheider ausgestattet wurde.

In dem Kolben wurden 312,8 Gramm (3,0 mol) Neopentylglykol und 966,9 Gramm (6,7 mol) 2-Ethylhexansäure sowie 1,0 Gew.-% Aktivkohle, bezogen auf den gesamten Reaktionsansatz, vorgelegt. Unter Rühren und unter Anlegen eines leichten Unterdrucks von 600 hPa wurde der Ansatz auf 200°C erhitzt und 2 Stunden bei dieser Temperatur belassen. Anschließend wurde der Druck schrittweise auf 500 hPa reduziert und gebildetes Reaktionswasser an dem Wasserabscheider abgenommen. Der Reaktionsverlauf wurde durch kontinuierliche Auswaage der über den Wasserabscheider ausgeschleusten Wassermenge sowie durch den Verlauf der Hydroxylzahl verfolgt. Nach insgesamt 8 Stunden Reaktionszeit wurde die Reaktion beendet.

Anschließend wurde über einen Zeitraum von 2 Stunden bei einer Temperatur von 190°C und einem Druck von 95 hPa und dann anschließend für weitere 0,5 Stunden bei einer Temperatur von 130°C und einem Druck von 6 hPa die überschüssige 2-Ethylhexansäure abdestilliert. Es folgte eine Wasserdampfdestillation über einen Zeitraum von 0,5 Stunden bei 180°C und bei Normaldruck und eine endgültige Trocknung bei einer Temperatur von 120°C über 15 Minuten. Nach abschließender Filtration von der Aktivkohle erhielt man hellfarbiges Neopentylglykol-di-2-ethylhexanoat mit folgenden Kennzahlen:

**Tabelle 2: Kennzahlen von Neopentylglykol-di-2-ethylhexanoat, hergestellt nach Beispiel 3**

| Gaschromatographische Analyse (Gew.-%): | |
|---|---|
| Neopentylglykol-di-2-ethylhexanoat | 93,2 % |
| Neopentylglykol-mono-2-ethylhexanoat | 5,8 % |
| Rest | 1,0 % |
| | |
| Farbzahl nach Hazen (DIN ISO 6271) | 69 |

### Beispiel 4 (Vergleichsbeispiel):

### Herstellung von Neopentylglykol-di-2-ethylhexanoat; Veresterung ohne Zusatz von Aktivkohle

Es wurde gemäß dem Ausführungsbeispiel 3 gearbeitet, jedoch wurde die Veresterungsreaktion ohne Zusatz von Aktivkohle durchgeführt. Da keine Feststoffe zugegen waren, wurde der Ansatz nach Veresterung und Aufarbeitung nicht filtriert.

**Tabelle 3: Kennzahlen von Neopentylglykol-di-2-ethylhexanoat, hergestellt nach Beispiel 4**

| Gaschromatographische Analyse (Gew.-%): | |
|---|---|
| Neopentylglykol-di-2-ethylhexanoat | 92,9 % |
| Neopentylglykol-mono-2-ethylhexanoat | 6,2 % |
| Rest | 0,9 % |
| | |
| Farbzahl nach Hazen (DIN ISO 6271) | 140 |

Auch diese Beispiele belegen, dass die Durchführung der Veresterungsreaktion in Gegenwart eines Adsorbens sich vorteilhaft auf die Farbzahl des gewünschten Polyolesters auswirkt.

## Patentansprüche

1. Verfahren zur Herstellung von Polyolestern durch Umsetzung von Polyolen der allgemeinen Formel
H-(-O-[-CR¹R²-]ₘ-)ₒ-OH
in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxylmethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten, mit linearen oder verzweigten aliphatischen Monocarbonsäuren mit 3 bis 20 Kohlenstoffatomen im Überschuss und anschließende Aufarbeitung des Reaktionsgemisches, **dadurch gekennzeichnet, dass** man eine Mischung der Ausgangsverbindungen in Gegenwart eines Adsorbens reagieren lässt, die unumgesetzten Ausgangsverbindungen abtrennt, anschließend eine Wasserdampfbehandlung bei einer Temperatur von 170 bis 220°C und über einen Zeitraum von 0,5 bis 5 Stunden durchführt und den zurückgebliebenen Polyolester trocknet, wobei nach beendeter Umsetzung oder nach einer beliebigen anderen Aufarbeitungsmaßnahme der Polyolester filtriert wird, mit der Maßgabe, dass die aliphatische Monocarbonsäure einen niedrigeren Siedepunkt als das eingesetzte Polyol aufweist und die Umsetzung ohne Einsatz eines Katalysators autokatalytisch durchgeführt wird.

2. Verfahren gemäß Anspruch 1, **dadurch gekennzeichnet, dass** man die Mischung der Ausgangsverbindungen in Gegenwart des Adsorbens auf eine Temperatur bis auf maximal 280°C, vorzugsweise bis auf 250°C, erhitzt und bei konstant gehaltener Temperatur den Druck von Stufe zu Stufe erniedrigt.

3. Verfahren gemäß Anspruch 1, **dadurch gekennzeichnet, dass** man die Mischung der Ausgangsverbindungen in Gegenwart des Adsorbens bei konstant eingestelltem Druck von Stufe zu Stufe bis auf eine maximale Temperatur von 280°C erhitzt.

4. Verfahren gemäß Anspruch 1, **dadurch gekennzeichnet, dass** man die Mischung der Ausgangsverbindungen in Gegenwart des Adsorbens bei von Stufe zu Stufe ansteigender Temperatur auf maximal 280°C erhitzt und auch den Druck von Stufe zu Stufe erniedrigt.

5. Verfahren gemäß Anspruch 4, **dadurch gekennzeichnet, dass** man eine Mischung der Ausgangsverbindungen in Gegenwart des Adsorbens in einer ersten Stufe bei einer Temperatur bis auf 190°C und bei einem Druck bis auf 600 hPa reagieren lässt und die Reaktion in einer zweiten Stufe durch Erhöhung der Temperatur bis auf 250°C und bei einem Druck bis auf 300hPa zu Ende führt.

6. Verfahren gemäß einem oder mehreren der Ansprüche 1 bis 5, **dadurch gekennzeichnet, dass** das Adsorbens in einer Menge von 0,05 bis 30, vorzugsweise von 0,1 bis 5,0 und insbesondere von 0,1 bis 1,0 Gew.-Teilen je 100 Gew.-Teilen flüssiger Phase eingesetzt wird.

7. Verfahren gemäß einem oder mehreren der Ansprüche 1 bis 6, **dadurch gekennzeichnet, dass** man als Adsorbens Silicagel, Kieselgel, Kieselguhr, Aluminiumoxid, Aluminiumoxidhydrate, Tone, Carbonate oder Aktivkohle verwendet.

8. Verfahren gemäß einem oder mehreren der Ansprüche 1 bis 7, **dadurch gekennzeichnet, dass** man die Wasserdampfbehandlung bei einer Temperatur 170 bis 200°C durchführt.

9. Verfahren gemäß einem oder mehreren der Ansprüche 1 bis 8, **dadurch gekennzeichnet, dass** der Polyolester nach der Wasserdampfbehandlung bei Temperaturen von 80 bis 250°C, vorzugsweise 100 bis 180°C und bei Drücken von 0,2 bis 500 hPa, vorzugsweise 1 bis 200 hPa und insbesondere von 1 bis 20 hPa getrocknet wird.

10. Verfahren gemäß Anspruch 9, **dadurch gekennzeichnet, dass** der Polyolester in Gegenwart eines Inertgases getrocknet wird.

11. Verfahren gemäß einem oder mehreren der Ansprüche 1 bis 10, **dadurch gekennzeichnet, dass** der Polyolester nach der Wasserdampfbehandlung filtriert wird.

12. Verfahren gemäß einem oder mehreren der Ansprüche 1 bis 10, **dadurch gekennzeichnet, dass** der Polyolester nach der Trocknung filtriert wird.

13. Verfahren gemäß einem oder mehreren der Ansprüche 1 bis 12, **dadurch gekennzeichnet, dass** man als Polyole Di-Trimethylolpropan, Di-Pentaerythrit, Diethylenglykol, Triethylenglykol, Tetraethylenglykol, Dipropylenglykol, Tripropylenglykol oder Tetrapropylenglykol verwendet.

14. Verfahren gemäß einem oder mehreren der Ansprüche 1 bis 13, **dadurch gekennzeichnet, dass** man als aliphatische Monocarbonsäure Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, 2-Ethylhexansäure, n-Nonansäure, 2-Methyloctansäure, Isononansäure, 3,5,5-Trimethylhexansäure oder 2-Propylheptansäure umsetzt.

15. Verfahren gemäß einem oder mehreren der Ansprüche 1 bis 14 zur Herstellung von Triethylenglykol-di-2-ethylhexanoat, Tetraethylenglykol-di-n-heptanoat, Triethylenglykol-di-2-ethylbutyrat,Tetraethylenglykol-di-2-ethylhexanoat oder Triethylenglykol-di-n-heptanoat.

## Claims

1. Process for preparing polyol esters by reacting polyols of the general formula
H-(-O-[-CR¹R²-]ₘ-)ₒ-OH
in which R¹ and R² are each independently hydrogen, an alkyl radical having 1 to 5 carbon atoms, preferably methyl, ethyl or propyl, or a hydroxyalkyl radical having 1 to 5 carbon atoms, preferably the hydroxymethyl radical, m is an integer of 1 to 10, preferably 1 to 8 and especially 1, 2, 3 or 4, o is an integer of 2 to 15, preferably 2 to 8 and especially 2, 3, 4 or 5, with linear or branched aliphatic monocarboxylic acids having 3 to 20 carbon atoms in excess and then working up the reaction mixture, **characterized in that** a mixture of the starting compounds is allowed to react in the presence of an adsorbent, the unconverted starting compounds are removed, then a steam treatment is performed at a temperature of 170 to 220°C and over a period of 0.5 to 5 hours and the remaining polyol ester is dried, the polyol ester being filtered after the reaction has ended or after any other workup measure with the proviso that the aliphatic monocarboxylic acid has a lower boiling point than the polyol used and the reaction is conducted autocatalytically without using a catalyst.

2. Process according to Claim 1, **characterized in that** the mixture of the starting compounds is heated in the presence of the adsorbent to a temperature up to a maximum of 280°C, preferably up to 250°C, and the pressure is lowered from stage to stage with the temperature kept constant.

3. Process according to Claim 1, **characterized in that** the mixture of the starting compounds is heated in the presence of the adsorbent at constant pressure from stage to stage up to a maximum temperature of 280°C.

4. Process according to Claim 1, **characterized in that** the mixture of the starting compounds is heated in the presence of the adsorbent at a temperature rising from stage to stage to a maximum of 280°C, and the pressure is also lowered from stage to stage.

5. Process according to Claim 4, **characterized in that** a mixture of the starting compounds is allowed to react in the presence of the adsorbent in a first stage at a temperature up to 190°C and at a pressure up to 600 hPa, and the reaction is conducted to completion in a second stage by increasing the temperature up to 250°C and at a pressure up to 300 hPa.

6. Process according to one or more of Claims 1 to 5, **characterized in that** the adsorbent is used in an amount of 0.05 to 30 parts, preferably of 0.1 to 5.0 parts and especially of 0.1 to 1.0 parts by weight per 100 parts by weight of liquid phase.

7. Process according to one or more of Claims 1 to 6, **characterized in that** the adsorbent used is silica gel, kieselguhr, aluminium oxide, aluminium oxide hydrates, clays, carbonates or activated carbon.

8. Process according to one or more of Claims 1 to 7, **characterized in that** the steam treatment is performed at a temperature of 170 to 200°C.

9. Process according to one or more of Claims 1 to 8, **characterized in that** the polyol ester, after the steam treatment, is dried at temperatures of 80 to 250°C, preferably 100 to 180°C, and at pressures of 0.2 to 500 hPa, preferably 1 to 200 hPa and especially of 1 to 20 hPa.

10. Process according to Claim 9, **characterized in that** the polyol ester is dried in the presence of an inert gas.

11. Process according to one or more of Claims 1 to 10, **characterized in that** the polyol ester is filtered after the steam treatment.

12. Process according to one or more of Claims 1 to 10, **characterized in that** the polyol ester is filtered after the drying.

13. Process according to one or more of Claims 1 to 12, **characterized in that** the polyols used are ditrimethylolpropane, dipentaerythritol, diethylene glycol, triethylene glycol, tetraethylene glycol, dipropylene glycol, tripropylene glycol or tetrapropylene glycol.

14. Process according to one or more of Claims 1 to 13, **characterized in that** the aliphatic monocarboxylic acid converted is propionic acid, n-butyric acid, isobutyric acid, n-pentanoic acid, 2-methylbutyric acid, 3-methylbutyric acid, 2-methylpentanoic acid, n-hexanoic acid, 2-ethylbutyric acid, n-heptanoic acid, 2-methylhexanoic acid, 2-ethylhexanoic acid, n-nonanoic acid, 2-methyloctanoic acid, isononanoic acid, 3,5,5-trimethylhexanoic acid or 2-propylheptanoic acid.

15. Process according to one or more of Claims 1 to 14 for preparing triethylene glycol di-2-ethylhexanoate, tetraethylene glycol di-n-heptanoate, triethylene glycol di-2-ethylbutyrate, tetraethylene glycol di-2-ethylhexanoate or triethylene glycol di-n-heptanoate.

## Revendications

1. Procédé pour la préparation de polyolesters par transformation de polyols de formule générale
H-(-O-[-CR¹R²-]ₘ-)ₒ-OH
dans laquelle R¹ et R² signifient, indépendamment l'un de l'autre, hydrogène, un radical alkyle comprenant 1 à 5 atomes de carbone, de préférence méthyle, éthyle ou propyle, ou un radical hydroxyalkyle comprenant 1 à 5 atomes de carbone, de préférence le radical hydroxyméthyle, m vaut un nombre entier de 1 à 10, de préférence de 1 à 8 et en particulier 1, 2, 3 ou 4, o vaut un nombre entier de 2 à 15, de préférence de 2 à 8 et en particulier 2, 3, 4 ou 5, avec des acides monocarboxyliques aliphatiques, linéaires ou ramifiés, comprenant 3 à 20 atomes de carbone en excès et traitement consécutif du mélange réactionnel, **caractérisé en ce qu'**on laisse réagir un mélange des composés de départ en présence d'un adsorbant, on sépare les composés de départ non transformé, on réalise ensuite un traitement à la vapeur d'eau à une température de 170 à 220 °C et sur un laps de temps de 0,5 à 5 heures et on sèche le polyolester qui reste, le polyolester étant filtré après la fin de la transformation ou après une quelconque autre mesure de traitement, à condition que l'acide monocarboxylique aliphatique présente un point d'ébullition inférieur à celui du polyol utilisé et que la transformation soit réalisée de manière autocatalytique sans utilisation d'un catalyseur.

2. Procédé selon la revendication 1, **caractérisé en ce qu'**on chauffe le mélange des composés de départ en présence de l'adsorbant à une température d'au maximum jusqu'à 280°C, de préférence jusqu'à 250°C, et on abaisse, en maintenant la température constante, la pression d'une étape à l'autre.

3. Procédé selon la revendication 1, **caractérisé en ce qu'**on chauffe le mélange des composés de départ en présence de l'adsorbant à une pression réglée à une valeur constante d'une étape à l'autre jusqu'à une température maximale de 280°C.

4. Procédé selon la revendication 1, **caractérisé en ce qu'**on chauffe un mélange des composés de départ en présence de l'adsorbant à une température augmentant d'une étape à l'autre jusqu'au maximum à 280°C et on abaisse également la pression d'une étape à l'autre.

5. Procédé selon la revendication 4, **caractérisé en ce qu'**on laisse réagir un mélange des composés de départ en présence de l'adsorbant, dans une première étape, à une température de jusqu'à 190°C et à une pression de jusqu'à 600 hPa et on conduit la réaction jusqu'à la fin dans une deuxième étape par augmentation de la température jusqu'à 250°C et à une pression jusqu'à 300 hPa.

6. Procédé selon l'une ou plusieurs des revendications 1 à 5, **caractérisé en ce que** l'adsorbant est utilisé en une quantité de 0,05 à 30, de préférence de 0,1 à 5,0 et en particulier de 0,1 à 1,0 partie (s) en poids par 100 parties en poids de phase liquide.

7. Procédé selon l'une ou plusieurs des revendications 1 à 6, **caractérisé en ce qu'**on utilise, comme adsorbant, le silicagel, le gel silicique, le kieselguhr, l'oxyde d'aluminium, l'oxyde d'aluminium hydraté, l'argile, des carbonates ou le charbon actif.

8. Procédé selon l'une ou plusieurs des revendications 1 à 7, **caractérisé en ce qu'**on réalise le traitement à la vapeur d'eau à une température de 170 à 200°C.

9. Procédé selon l'une ou plusieurs des revendications 1 à 8, **caractérisé en ce que** le polyolester est séché après le traitement à la vapeur d'eau à des températures de 80 à 250°C, de préférence de 100 à 180°C et à des pressions de 0,2 à 500 hPa, de préférence de 1 à 200 hPa et en particulier de 1 à 20 hPa.

10. Procédé selon la revendication 9, **caractérisé en ce que** le polyolester est séché en présence d'un gaz inerte.

11. Procédé selon l'une ou plusieurs des revendications 1 à 10, **caractérisé en ce que** le polyolester est filtré après le traitement à la vapeur d'eau.

12. Procédé selon l'une ou plusieurs des revendications 1 à 10, **caractérisé en ce que** le polyolester est filtré après le séchage.

13. Procédé selon l'une ou plusieurs des revendications 1 à 12, **caractérisé en ce qu'**on utilise, comme polyols, le di-triméthylolpropane, le di-pentaérythritol, le diéthylèneglycol, le triéthylèneglycol, le tétraéthylèneglycol, le dipropylèneglycol, le tripropylèneglycol ou le tétrapropylèneglycol.

14. Procédé selon l'une ou plusieurs des revendications 1 à 13, **caractérisé en ce qu'**on utilise comme acide monocarboxylique aliphatique les acides propionique, n-butyrique, isobutyrique, n-pentanoïque, 2-méthylbutyrique, 3-méthylbutyrique, 2-méthylpentanoïque, n-hexanoïque, 2-éthylbutyrique, n-heptanoïque, 2-méthylhexanoïque, 2-éthylhexanoïque, n-nonanoïque, 2-méthyloctanoïque, isononanoïque, 3,5,5-triméthylhexanoïque ou 2-propylheptanoïque.

15. Procédé selon l'une ou plusieurs des revendications 1 à 14 pour la préparation de di-2-éthylhexanoate de triéthylèneglycol, de di-n-heptanoate de tétraéthylèneglycol, de di-2-éthylbutyrate de triéthylèneglycol, de di-2-éthylhexanoate de tétraéthylèneglycol ou de di-n-heptanoate de triéthylèneglycol.
